# EUROPEAN PATENT APPLICATION

(11) **EP 4 620 505 A1**
(43) Date of publication of application: **24.09.2025**
(21) Application number: 23891326.3
(22) Date of filing: 27.10.2023
(51) Int. Cl.: A61M 25/09

(54) **GUIDE WIRE**

(30) Priority: 16.11.2022 JP 2022183146
(71) Applicant: ASAHI INTECC CO., LTD., Seto-shi, Aichi, 489-0071 (JP)
(72) Inventor: TAKAI Ami, Seto-shi, Aichi 489-0071 (JP); NAGAO Narumi, Seto-shi, Aichi 489-0071 (JP)
(74) Representative: Winter, Brandl - Partnerschaft mbB
(86) International application number: PCT/JP2023/038826
(87) International publication number: WO 2024/106172

(57) **Abstract**

A guide wire 10 includes an elongated core 1, and a cylindrical body 2 disposed outside the core 1, in which an outer peripheral surface of the cylindrical body 2 is partitioned into a first region 21 located on a distal end side and a second region 22 located on a proximal end side with respect to the first region 21, and the first region 21 has a surface property different from a surface property of the second region 22. The guide wire 10 may configured such that a first covering layer 6 is formed on a surface of the first region 21, and a second covering layer 7 is formed on a surface of the second region 22. The guide wire 10 makes it possible to suppress progression of a prolapse at any position in spite of a simple structure.

## Description

### TECHNICAL FIELD

The disclosed embodiments relate to a guide wire.

### BACKGROUND ART

Guide wires have been conventionally used for guiding, to a target site, a catheter-like medical instrument that is inserted into a tubular organ of a human body, such as a blood vessel or a digestive organ.

There is a procedure in which, when a device such as a catheter and an internal indwelling instrument is guided to a target site in a blood vessel using a guide wire, the guide wire is pushed forward in the blood vessel while the distal end of the guide wire is folded back and bent into a U-shape (prolapsing shape) to prevent a damage (perforation) of a blood vessel wall due to the distal end of the guide wire. At this time, if a progression length of the prolapse is extremely elongated, a reaction force caused by the bending of the guide wire increases, and the force of pushing the guide wire must be strengthened, but there is a concern that the stronger the pushing force is, the more likely the blood vessels are damaged.

Patent Literature 1 discloses a technique in which, for the purpose of suppressing the progression of the prolapse at a desired position on the distal end portion of the guide wire, a rigidity difference is provided in a longitudinal direction of a guide wire so that the prolapse can be prevented from progressing to a rear end side with respect to the position of the rigidity gap. Specifically, the rigidity difference is provided in a longitudinal direction of the guide wire by factors that e.g. a coil surrounding the outer periphery of the core shaft has a double structure of an outer flexible tube body and an inner flexible tube body on the distal end portion of the guide wire and a distal end of the inner flexible tube body deviates to a proximal end side with respect to a distal end of the outer flexible tube body, a joint part having a high rigidity is partially formed, and the inner flexible tube body is processed into a tapered shape with an outer diameter decreasing toward the distal end.

### CITATION LIST

### Patent Literature

Patent Literature 1: JP 2012-055731 A

### SUMMARY OF INVENTION

### TECHNICAL PROBLEM

However, there have been problems that, when a rigidity difference is structurally provided as in the guide wire disclosed in Patent Literature 1, the guide wire tends to break (fracture) at a portion of the rigidity difference, i.e. at a physical rigidity change point. Also, there have been problems that the structure causing such a rigidity difference is relatively complicated, and manufacturing burdens are high, such as increased numbers of components and processes and an increased assembly cost.

The disclosed embodiments have been made in view of the above circumstances, and an object of the disclosed embodiments is to provide a guide wire capable of suppressing progression of prolapse at any position in spite of a simple structure.

### SOLUTION TO PROBLEM

In order to achieve the above object, the disclosed embodiments provide a guide wire including an elongated core and a cylindrical body disposed outside the core, in which an outer peripheral surface of the cylindrical body is partitioned into a first region located on the distal end side and a second region located on the proximal end side with respect to the first region, and the first region has a surface property different from a surface property of the second region (Invention 1).

According to this disclosed embodiment (Invention 1), in spite of a simple structure, progression of prolapse can be suppressed at any position depending on the surface properties of the first region and the second region.

### BRIEF DESCRIPTION OF DRAWINGS

FIG. 1 is an explanatory view illustrating a structure of a guide wire according to an embodiment of the disclosed embodiments.
FIG. 2 is an explanatory view illustrating a structure of a guide wire in Modification 1.
FIG. 3 is an explanatory view illustrating a structure of a guide wire in Modification 2.
FIG. 4 is an explanatory view illustrating a structure of a guide wire in Modification 3.
FIG. 5 is an explanatory view illustrating a structure of a guide wire in Modification 4.

### EMBODIMENTS OF THE INVENTION

A guide wire 10 according to the disclosed embodiments will be explained below with reference to the drawings. The guide wire 10 is a medical device that is used for inserting a catheter into a blood vessel, a digestive organ, or the like. A distal end side of the guide wire 10 is a side to be inserted into a body, and a proximal end side of the guide wire 10 is a side to be operated by a professional such as a surgeon. The disclosed embodiments are not limited to the embodiments explained below, and the described embodiments are merely examples for explaining the technical features of the disclosed embodiments. The shapes and dimensions illustrated in the drawings are merely illustrated to facilitate understanding of the contents of the disclosed embodiments, and do not accurately reflect actual shapes and dimensions.

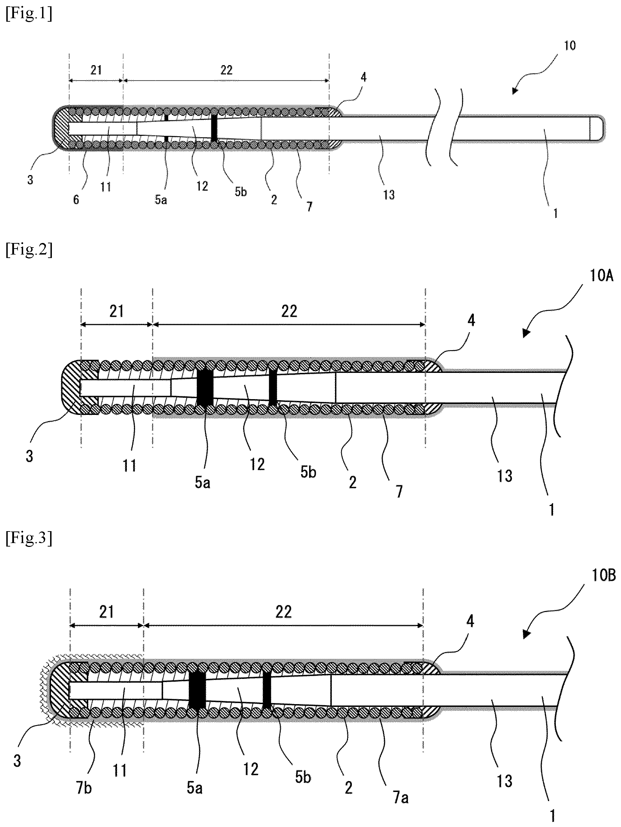

In this specification, the "distal end side" means one side of a direction along an axial direction of the guide wire, in which the guide wire progresses toward a target site. The "proximal end side" means one side of a direction along the axial direction of the guide wire, which is opposite to the distal end side. The "distal end" refers to an end portion on a distal end side of any member or site, and the "proximal end" refers to an end portion on a proximal end side of any member or site. The "distal end portion" refers to a part including the distal end of any member or site and extending from the distal end toward the proximal end side up to the middle of the member or the like, and the "proximal end portion" refers to a part including the proximal end of any member or site and extending from the proximal end toward the distal end side up to the middle of the member or the like. In FIG. 1, the left side in the drawing is the "distal end side" to be inserted into a body, and the right side in the drawing is the "proximal end side" to be operated by a professional.

FIG. 1 is an explanatory view illustrating a structure of the guide wire 10 according to the present embodiment. The guide wire 10 includes an elongated core 1 and a cylindrical body 2 provided outside the core 1. A distal tip 3 for joining the core 1 and the cylindrical body 2 is provided on the distal end of the guide wire 10, and a fixation portion 4 for fixing the core 1 and the cylindrical body 2 is provided on a proximal end of the cylindrical body 2.

The core 1 is an elongated member serving as a shaft of the guide wire 10. As illustrated in FIG. 1, the core 1 has a small diameter portion 11 on the distal end side and a large diameter portion 13 on the proximal end side, and has a tapered portion 12 disposed between the small diameter portion 11 and the large diameter portion 13 and having an outer diameter decreasing from the proximal end side toward the distal end side. The core 1 can be formed of, for example, materials such as stainless alloys (SUS302, SUS304, SUS316, etc.), superelastic alloys such as an Ni-Ti alloy, a piano wire, a nickel-chromium alloy, a cobalt alloy, and tungsten. The material for the core 1 is not limited these materials, and the core 1 may be formed of other known materials as long as the core 1 itself can be prevented from being cut and has a rotatable distal end portion.

The small diameter portion 11, the tapered portion 12, and the large diameter portion 13 of the core 1 have different rigidities because of the difference in diameter. The small diameter portion 11 of the core 1 is an example of a first rigid portion in the disclosed embodiments, and the tapered portion 12 and the large diameter portion 13 of the core 1 are examples of a second rigid portion.

The small diameter portion 11 has a columnar shape with an outer diameter that is constant from the distal end to the proximal end, and has a sectional secondary moment that is constant throughout the small diameter portion 11. The large diameter portion 13 also has a columnar shape with an outer diameter constant from the distal end to the proximal end, and has a sectional secondary moment constant throughout the large diameter portion 13. The tapered portion 12 has a circular truncated cone shape with an outer diameter gradually increasing from the distal end toward the proximal end, and also has a sectional secondary moment gradually increasing from the distal end toward the proximal end.

The cylindrical body 2 is wound around the core 1 so as to cover the outer peripheries of the small diameter portion 11, the tapered portion 12, and a part of the large diameter portion 13 of core 1. That means, the cylindrical body 2 is arranged so as to straddle between the small diameter portion 11 as the first rigid portion of the core 1 and the tapered portion 12 and large diameter portion 13 as the second rigid portions of core 1.

The cylindrical body 2 may be a single coil formed into a hollow cylindrical shape by spirally winding one wire with a circular cross-section or may be a hollow twisted wire coil formed into a hollow cylindrical shape using a twisted wire with a plurality of wires twisted. The cylindrical body 2 may be composed of a combination of a single coil and a hollow twisted wire coil. The cylindrical body 2 can be formed of, for example, stainless alloys (SUS302, SUS304, SUS316, etc.), superelastic alloys such as an Ni-Ti alloy, a piano wire, a nickel-chromium alloy, radiotransparent alloys such as a cobalt alloy, radiopaque alloys such as gold, platinum, tungsten, and an alloy containing these elements (e.g. a platinum-nickel alloy), but the material is not limited to these examples, and the cylindrical body 2 may be formed of known materials other than the above-described materials. In the present embodiment, the cylindrical body 2 is formed as a single member wholly made of same materials, and configured to have an outer diameter constant from the distal end to the proximal end.

The distal tip 3 for joining the core 1 with the cylindrical body 2 is formed on the distal end of the guide wire 10 (i.e. the distal end of the core 1). The distal tip 3 is formed of a metal solder such as silver braze, gold braze, zinc, an Sn-Ag alloy, and an Au-Sn alloy, and the distal end of the core shaft 1 and the distal end of the cylindrical body 2 are fixed to each other by the metal solder. The distal tip 3 may be formed of an adhesive such as an epoxy adhesive so that the distal end of the core 1 and the distal end of the cylindrical body 2 are fixed to each other by the adhesive.

The fixation portion 4 for fixing the core 1 with the cylindrical body 2 is formed on the proximal end of the cylindrical body 2. The fixation portion 4 is formed of a metal solder such as silver braze, gold braze, zinc, an Sn-Ag alloy, and an Au-Sn alloy, and the proximal end of the cylindrical body 2 is fixed to the large diameter portion 13 of the core 1 by this metal solder. The fixation portion 4 may be formed of an adhesive such as an epoxy adhesive so that the large diameter portion 13 of the core 1 and the proximal end of the cylindrical body 2 are fixed to each other by the adhesive.

Two joint parts 5a and 5b for joining the tapered portion 12 of the core 1 with the cylindrical body 2 are formed inside the cylindrical body 2. The joint parts 5a and 5b are formed of a metal solder such as silver braze, gold braze, zinc, an Sn-Ag alloy, and an Au-Sn alloy, and the tapered portion 12 of the core 1 and the cylindrical body 2 are fixed to each other by this metal solder. The joint parts 5a and 5b may be formed of an adhesive such as an epoxy adhesive so that the tapered portion 12 of the core 1 and the cylindrical body 2 are fixed to each other by the adhesive.

An outer peripheral surface of the cylindrical body 2 is partitioned into a first region 21 located on the distal end side and a second region 22 located on the proximal end side with respect to the first region 21, and the first region 21 has a surface property different from a surface property of the second region 22. Specifically, the cylindrical body 2 is configured so that the first region 21 has a surface friction coefficient µ₁ larger than a surface friction coefficient µ₂ of the second region 22. In other words, the surface friction coefficient µ₁ of the first region 21 and the surface friction coefficient µ₂ of the second region 22 satisfy a relational expression µ₁>µ₂. As described later, since a boundary between the first region 21 and the second region 22 serves as a reference position for determining to what extent the prolapse is allowed to progress in the guide wire 10, the lengths of the first region 21 and the second region 22 can be arbitrarily determined depending on the intended purpose of the guide wire 10, or the like.

The relationship between the surface friction coefficient µ₁ of the first region 21 and the surface friction coefficient µ₂ of the second region 22 may be adjusted e.g. by forming covering layers made of different materials on the both regions, or by forming a covering layer only on one region, or by performing different surface treatments on the both regions, or by forming covering layers made of same materials on the both regions and then performing a surface treatment only on a covering layer formed on one region.

In the present embodiment, a silicone coating layer 6 is formed on the surfaces of the first region 21 of the cylindrical body 2 and the distal tip 3, and a hydrophilic coating layer is formed on the surfaces of the second region 22 of the cylindrical body 2, the fixation portion 4, and the proximal end portion of the large diameter portion 13 of the core 1. Materials for the silicone coating layer 6 and the hydrophilic coating layer are selected so that the surface friction coefficient µ₁ of the silicone coating layer 6 is larger than the surface friction coefficient µ₂ of the hydrophilic coating layer.

The silicone coating layer 6 is an example of a first covering layer in the disclosed embodiments and can be formed by a known coating forming method, e.g. by applying a medical-grade silicone solution on the surfaces of the first region 21 of the cylindrical body 2 and the distal tip 3.

The hydrophilic coating layer 7 is an example of a second covering layer in the disclosed embodiments and can be formed e.g. by a known coating forming method, in which a solution including a nonionic hydrophilic polymer such as polyvinyl alcohol, polyvinylpyrrolidone, polyethylene glycol, polyacrylamide, polymethylacrylamide, poly(2-hydroxyethyl methacrylate), and poly(N-hydroxyethylacrylamide); an anionic hydrophilic polymer such as polyacrylic acid, polymethacrylic acid, polymaleic acid, carboxymethyl cellulose, hyaluronic acid, and poly(2-acrylamide-2-methylpropanesulfonic acid); or a cationic hydrophilic polymer such as polyethyleneimine, polyallylamine, and polyvinylamine is applied on the surfaces of the second region 22 of the cylindrical body 2, the fixation portion 4, and the proximal end side of the large diameter portion 13 of the core 1.

According to this guide wire 10, on the surface of the cylindrical body 2j provided outside the core 1, by providing the first region 21 as the high friction region on the distal end side and the second region 22 as the low friction region on the proximal end side, the guide wire 10 becomes easier to bend up to the vicinity of the boundary between the first region 21 and the second region 22 to achieve a guide wire with the prolapse suppressed from progressing toward the proximal end side with respect to the vicinity or the boundary. Since this guide wire 10 makes it possible to arbitrarily decide how to arrange the first region 21 and the second region 22 on the surface of the cylindrical body 2, there is no longer any need to make the structure of the guide wire complex to generate a rigidity gap as in the past, and therefore the progression of the prolapse can be suppressed at any position in spite of the simple structure.

In particular, in the guide wire 10 according to the present embodiment, the boundary between the first region 21 located on the distal end side and the second region 22 located on the proximal end side with respect to the first region 21 on the outer peripheral surface of the cylindrical body 2, is located on the outer peripheral surface of the small diameter portion 11 (first rigid portion) of the core 1. The small diameter portion 11 is formed so as to have an outer diameter that is constant from the distal end to the proximal end and have a sectional secondary moment that is constant throughout the small diameter portion 11, and therefore the possibility of the guide wire 10 to be broken due to the rigidity difference on the core 1 can be reduced even if the guide wire 10 is bent in the vicinity of the boundary between the first region 21 and the second region 22.

The guide wire according to the disclosed embodiments has been explained above with reference to the drawings, but the disclosed embodiments are not limited to the above embodiment and can be variously modified. For example, in the above embodiment, the case of the core having the large diameter portion on the proximal end side, the small diameter portion on the distal end side, and the tapered portion located between the large diameter portion and the small diameter portion, has been explained as an example. However, the core used for the guide wire according to the disclosed embodiments may have an outer diameter that is constant from the proximal end side to the distal end side.

In the above embodiment, as an example of the first rigid portion according to the disclosed embodiments, the columnar small diameter portion 11 with an outer diameter that is constant from the distal end to the proximal end is disposed on the distal end side of the core 1, but the core is not limited to this shape, and for example, the first rigid portion may have a distal end portion, a proximal end portion having a cross-sectional shape different from that of the distal end portion, and a transition portion having a cross-sectional shape transitioning from the distal end portion to the proximal end portion. As an example, the distal end portion may have a flat cross-sectional shape, the proximal end portion may have a circular cross-sectional shape, and the transition portion may have a cross-sectional shape transitioning from a flat shape to a circular shape.

When the core has a first rigid portion with the distal end portion, the transition portion, and the proximal end portion as described above, the covering boundary between the first covering layer (e.g. silicone coating layer) and the second covering layer (e.g. hydrophilic coating layer) formed on the outer peripheral surface of the cylindrical body may be located on the distal end portion or the proximal end portion of the core, or on the transition portion of the core.

Modifications of the guide wire 1 described in the above embodiment will be explained below with reference to FIG. 2 to FIG. 5. In the guide wire of each modification illustrated in FIG. 2 to FIG. 5, the proximal end side is omitted from the illustration. In the guide wire of each modification, the same components as for the guide wire 1 are marked with the same symbols as for the guide wire 1, and their descriptions are omitted.

### <Modification 1>

FIG. 2 is an explanatory view illustrating a structure of Modification 1 of the guide wire according to the disclosed embodiments. A guide wire 10A of Modification 1 is different from the above-described guide wire 10 in that the guide wire 10A does not have the silicone coating layer (first covering layer) covering the first region 21 and the distal tip 3.

Even if the first region 21 is not covered, as long as the first region 21 has a surface friction coefficient larger than that of the second region 22 covered with the hydrophilic coating layer (second covering layer), the guide wire 10A can be easily bent up to the vicinity of the boundary between the first region 21 and the second region 22 to achieve a guide wire with a prolapse suppressed from progressing toward the proximal end side with respect to the vicinity of the boundary.

### <Modification 2>

FIG. 3 is an explanatory view illustrating a structure of Modification 2 of the guide wire according to the disclosed embodiments. In a guide wire 10B of Modification 2, both the first region 21 and the second region 22 are covered with a same hydrophilic coating layer, but the surface of only the hydrophilic coating layer that covers the first region 21 is processed so as to increase the friction coefficient, and in this regard, the guide wire 10B is different from the guide wire 10. Specifically, in the guide wire 10B, the surfaces of the second region 22, the fixation portion 4, and the core 1 on the proximal end side are covered with a hydrophilic coating layer 7a and the first region 21 and the distal tip 3 are coated with hydrophilic coating layer 7b, and irregularities are formed on the surface of the hydrophilic coating layer 7a that covers the first region 21 e.g. by blasting, to increase the surface friction coefficient of the hydrophilic coating layer 7a.

Even if the first region 21 and the second region 22 are covered with a covering layer made of a same material, as long as the first region 21 has a surface friction coefficient larger than that of the second region 22 by surface treatment or the like, the guide wire 10B can be easily bent up to the vicinity of the boundary between the first region 21 and the second region 22 to achieve a guide wire with a prolapse suppressed from progressing toward the proximal end side with respect to the vicinity of the boundary.

### <Modification 3>

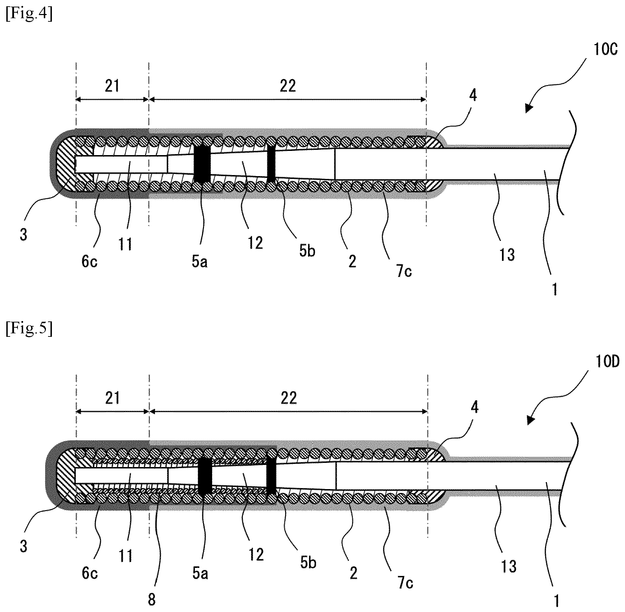

FIG. 4 is an explanatory view illustrating a structure of Modification 3 of the guide wire according to the disclosed embodiments. In a guide wire 10C of Modification 3, a silicone coating layer 6c formed on the surface of the first region 21 is also formed on the second region 22 side beyond the boundary between the first region 21 and the second region 22, a hydrophilic coating layer 7c formed on the surface of the second region 22 is formed on the top of the silicone coating layer 6c formed on the second region 22 side, and in this regard, the guide wire 10C is different from the guide wire 10. That means, in the guide wire 10C, the proximal end portion of the silicone coating layer 6c covering the first region 21 enters under the hydrophilic coating layer 7c covering the second region 22, so as to extend between the hydrophilic coating layer 7c covering the second region 22 and the cylindrical body 2.

Even if the silicone coating layer 6c covering the first region 21 extends under the hydrophilic coating layer 7c covering the second region 22, the location of the silicone coating layer 6c on the outermost side of the first region 21 and the location of the hydrophilic coating layer 7c on the outermost side of the second region 22 are not affected. Thus, since the surface friction coefficient of the first region 21 is still larger than that of the second region 22, the guide wire 10C can be easily bent up to the vicinity of the boundary between the first region 21 and the second region 22 to achieve a guide wire with a prolapse suppressed from progressing toward the proximal end side with respect to the vicinity of the boundary.

On the distal end side of the second region 22, the silicone coating layer 6c is formed under the hydrophilic coating layer 7c, so that the guide wire 10C can be prevented from becoming too stiff by the hydrophilic coating layer 7c, and rapid change in the rigidity in the vicinity of the boundary between the first region 21 and the second region 22 can be alleviated.

### <Modification 4>

FIG. 5 is an explanatory view illustrating a structure of Modification 4 of the guide wire according to the disclosed embodiments. In a guide wire 10D of Modification 4, an inner cylindrical body 8 is disposed inside the cylindrical body 2 along the outer periphery of the core 1, the silicone coating layer 6c formed on the surface of the first region 21 is also formed on the second region 22 side beyond the boundary between the first region 21 and the second region 22, the hydrophilic coating layer 7c formed on the surface of the second region 22 is formed on top of the silicone coating layer 6c formed on the second region 22 side, and in this regard, the guide wire 10D is different from the guide wire 10. Specifically, the inner cylindrical body 8 shorter than the cylindrical body 2 is wound around the outside of the core 1 so as to cover the outer periphery of the core 1 from the small diameter portion 11 to a part of the tapered portion 12, and the cylindrical body 2 and the inner cylindrical body 8 overlap with each other on the outside of the core 1 only on the distal end portion of the guide wire 10D. The distal end of the inner cylindrical body 8 is fixed to the distal tip 3, and the proximal end of the inner cylindrical body 8 is fixed to the joint part 5b. The silicone coating layer 6c formed on the outer surface of the cylindrical body 2 from the first region 21 to the second region 22 extends to the vicinity of the proximal end (joint part 5b) of the inner cylindrical body 8.

The inner cylindrical body 8 may be a single coil formed into a hollow cylindrical shape by spirally winding one wire with a circular cross-section or may be a hollow twisted wire coil formed into a hollow cylindrical shape using a twisted wire with a plurality of wires twisted. The inner cylindrical body 8 may be composed of a combination of a single coil and a hollow twisted wire coil. The inner cylindrical body 8 can be formed of, for example, stainless alloys (SUS302, SUS304, SUS316, etc.), superelastic alloys such as an Ni-Ti alloy, a piano wire, a nickel-chromium alloy, radiotransparent alloys such as a cobalt alloy, radiopaque alloys such as gold, platinum, tungsten, and an alloy containing these elements (e.g. a platinum-nickel alloy), but the material is not limited to these examples, and the inner cylindrical body 8 may be formed of known materials other than the above-described materials. In this modification, the inner cylindrical body 8 is formed as a single member wholly made of same materials, and configured to have an outer diameter constant from the distal end to the proximal end.

As described above, even if the cylindrical body disposed on the outside of the core 1 has a double structure, as long as, on the surface of the outer cylindrical body (i.e. cylindrical body 2), the first region 21 as the high friction region is disposed on the distal end side and the second region 22 as the low friction region is disposed on the proximal end side, the guide wire 10 can be easily bent up to the vicinity of the boundary between the first region 21 and the second region 22 to achieve a guide wire with the prolapse prevented from progressing to the proximal end side with respect to the vicinity of the boundary. The rigidity of the distal end portion of the guide wire 10D can be controlled by adjusting the structure and the length of the inner cylindrical body 8 disposed inside the cylindrical body 2.

### DESCRIPTION OF REFERENCE NUMERALS

10: Guide wire
1: Core
11: Small diameter portion
12: Tapered portion
13: Large diameter portion
2: Cylindrical body
21: First region
22: Second region
3: Distal tip
4: Fixation portion
5a, 5b: Joint part
6: Silicone coating layer (first covering layer)
7: Hydrophilic coating layer (second covering layer)
8: Inner cylindrical body

## Claims

1. A guide wire comprising: an elongated core; and
a cylindrical body disposed outside the core, wherein
an outer peripheral surface of the cylindrical body is partitioned into a first region located on a distal end side and a second region located on a proximal end side with respect to the first region, and
the first region has a surface property different from a surface property of the second region.

2. The guide wire according to claim 1, wherein the core has at least a first rigid portion and a second rigid portion having different rigidities, and
the cylindrical body is arranged so as to straddle between the first rigid portion and the second rigid portion.

3. The guide wire according to claim 2, wherein a boundary between the first region and the second region is located on an outer peripheral surface of the first rigid portion.

4. The guide wire according to claim 2 or 3, wherein the first rigid portion has a sectional secondary moment that is constant throughout the first rigid portion.

5. The guide wire according to claim 2 or 3, wherein a first covering layer is formed on a surface of the first region, and a second covering layer is formed on a surface of the second region, and
the first rigid portion has a distal end portion, a proximal end portion having a cross-sectional shape outer diameter different from that of the distal end portion, and a transition portion having a cross-sectional shape transitioning from the distal end portion to the proximal end portion.

6. The guide wire according to claim 5, wherein the distal end portion has a flat cross-sectional shape.

7. The guide wire according to claim 5 or 6, wherein a covering boundary between the first covering layer and the second covering layer is located on the distal end portion or the proximal end portion.

8. The guide wire according to claim 5 or 6, wherein a covering boundary between the first covering layer and the second covering layer is located on the transition portion.

9. The guide wire according to any one of claims 1 to 4, wherein a first covering layer is formed on a surface of the first region, and a second covering layer is formed on a surface of the second region.

10. The guide wire according to any one of claims 5 to 9, wherein a proximal end portion of the first covering layer extends between the second covering layer and the cylindrical body.

11. The guide wire according to any one of claims 1 to 10, wherein an inner cylindrical body is disposed inside the cylindrical body along an outer periphery of the core.

12. The guide wire according to any one of claims 1 to 11, wherein the first region has a surface friction coefficient larger than a surface friction coefficient of the second region.
